# EUROPEAN PATENT APPLICATION

(11) **EP 3 272 768 A1**
(43) Date of publication of application: **24.01.2018**
(21) Application number: 16305952.0
(22) Date of filing: 22.07.2016
(51) Int. Cl.: C07K 14/445, A61K 38/17

(54) **CHIMERIC PEPTIDES USEFUL IN MALARIA PROPHYLAXIS OR TREATMENT**

(71) Applicant: Université Pierre et Marie Curie (Paris 6), 75005 Paris (FR); Centre National de la Recherche Scientifique, 75016 Paris (FR); INSERM (Institut National de la Santé et de la Recherche Médicale), 75013 Paris (FR)
(72) Inventor: REBOLLO GARCIA, Angelita, 75015 Paris (FR); PIERROT, Christine, 59930 La Chapelle d'Armentières (FR); KHALIFE, Jamal, 59130 Lambersart (FR); ZHANGUI, Gigliola, 75002 Paris (FR)
(74) Representative: Cabinet Becker et Associés

(57) **Abstract**

The invention relates to a chimeric peptide construct comprising a cell penetrating peptide linked to a peptide that binds a protein of Plasmodium, especially Protein Phosphatase 1 (PP1). The construct is useful in preventing or treating malaria.

## Description

### Background of the invention

Malaria is a parasitic disease caused by a protozoon of the genus Plasmodium. The five subspecies active in humans are *P. falciparum, P. vivax, P. malariae, P. ovale,* and *P. knowlesi* with *P. falciparum* being responsible for most malaria-related death. *P. falciparum* is the most prevalent malaria parasite on the African continent. *P. vivax* is the dominant malaria parasite in most countries outside of sub-Saharan Africa. The malaria parasite is transmitted by the bites of the female mosquitoes of the genus Anopheles, especially *Anopheles gambiae.* The life cycle of all species of human malaria parasites is essentially the same. It comprises an exogenous sexual phase (sporogony) with multiplication in certain Anopheles mosquitoes and an endogenous asexual phase (schizogony) with multiplication in the vertebrate host.

During a blood meal, a malaria-infected female *Anopheles* mosquito inoculates sporozoites into the human host. Sporozoites infect liver cells and mature into schizonts, which rupture and release merozoites. In *P. vivax* and *P. ovale* a dormant stage (hypnozoites) can persist in the liver and cause relapses by invading the bloodstream weeks, or even years later. After this initial replication of the parasite in the liver (exo-erythrocytic schizogony), merozoites infect red blood cells and the parasites undergo asexual multiplication in the erythrocytes (erythrocytic schizogony). The ring stage trophozoites mature into schizonts. When fully developed, the schizont ruptures the red blood cell releasing the merozoites into the circulation. These merozoites will then infect new red cells and the process of asexual reproduction in the blood tends to proceed. This erythrocytic cycle of schizogony is repeated over and over again in the course of infection, leading to a progressive increase of parasitemia and resulting in the clinical manifestation of the disease. Some of the merozoites entering red blood cells do not form trophozoites and then schizonts but develop into gametocytes. These gametocytes make it possible for the cycle to continue in the *Anopheles* mosquito.

The malaria prevention rests on two types of measures, the preventive treatment with certain drugs and a set of precautions aiming at limiting the risks of infections. Use of prophylactic drugs is seldom practical for full-time residents of malaria-endemic areas, and their use is usually restricted to short-term visitors and travelers to malarial regions. This is due to the cost of purchasing the drugs, negative side effects from long-term use, and because some effective antimalarial drugs are difficult to obtain in certain countries.

Treatment and prophylaxis of malaria are complicated by the occurrence of the resistance of anopheles mosquitoes to insecticides and the growing resistance of Plasmodium strains to antimalarial drugs.

Facing chloroquine-resistance of *P. falciparum,* the World Health Organization recommended in 2001 artemisinin-based combination therapies (ACTs) for the treatment of uncomplicated falciparum malaria in nearly all areas. ACTs consist of a potent artemisinin component, which rapidly clears most parasites, plus a longer acting partner drug, which eliminates remaining parasites and limits selection of artemisinin resistance. Despite a considerable effort to make these ACTs affordable for the populations in question, their cost still remains high. Predictably, *P. falciparum* resistance to ACTs has been recently detected in 5 countries of the Greater Mekong Subregion. There are concerns that multi-drug resistant P. *falciparum* malaria could spread to other regions with direct public health consequences (Cui et al., 2015).

Infection by *P. vivax* poses unique challenges for diagnosis and treatment. Relatively low numbers of parasites in peripheral circulation may be difficult to confirm, and patients infected by dormant liver stages cannot be diagnosed before activation and the ensuing relapse. Radical cure thus requires therapy aimed at both the blood stages of the parasite (blood schizontocidal) and prevention of subsequent relapses (hypnozoitocidal). Chloroquine and primaquine have been the companion therapies of choice for the treatment of vivax malaria since the 1950s. Confirmed resistance to chloroquine occurs in much of the vivax endemic world (Baird et al, 2013).

Therefore, appropriate antimalarial prophylaxis and treatment are mainly guided by the infecting Plasmodium species and the drug susceptibility of the infecting parasites as determined by the geographic area where the infection is at risk or was acquired.

At present, it therefore remains essential to develop novel anti-malarial drugs active during the blood as well as tissue stages, without any or weak toxicity.

One of the major obstacles in devising new control tools is the limited knowledge of basic parasite biology and the paucity of identified potential intervention targets. In this context, the exploration of protein-protein interactions as new antimalarial drug targets was presented as an attractive area of research.

P. falciparum Protein Phosphatase 1 (PfPP1) is a plasmodial phosphatase playing a role in a high diversity of biological functions such as protein folding/proteolysis, transcription and pathogenicity. To date, 186 PfPP1 interacting proteins susceptible to regulate PfPP1 activity have been identified (Hollin et al., 2016). Although little is known about the regulation of PfPP1, biochemical and structure-activity relationship studies demonstrated that *P. falciparum* Leucine Rich Repeat 1 (PfLRR1) and 7 (PfLRR7) proteins exhibit an inhibitory role on PfPP1 activity (Daher et al., 2006). For these reasons, PfLRR proteins have been proposed as targets for drug discovery (Pierrot et al., 2012).

### Summary of the invention

The inventors have now mapped the binding site between *P. falciparum* Leucine Rich Repeat 1 (LRR1 or PfLRR1) protein and PP1 (or PfPP1) protein. On this basis they designed chimeric peptide constructs which are useful in preventing or treating malaria.

The invention more particularly provides a chimeric peptide construct comprising a chimeric peptide construct comprising a cell penetrating peptide linked to a peptide that binds a protein of Plasmodium, wherein said cell-penetrating peptide is X₁-KKKIK-**Ψ**-EI-X₂-X₃ (SEQ ID NO:1)
wherein X₁ is vacant, is a lysine residue, or valine-lysine;
X₂ is vacant, is a lysine residue, or lysine-isoleucine;
X₃ is vacant or is an amino acid sequence of one to 4 amino acids;
and Ψ is any amino-acid;
or a proteolysis-resistant peptide deriving from SEQ ID NO: 1 by one or more chemical modifications, or a substantially homologous peptide deriving from SEQ ID NO:1 by one or more conservative substitutions;
and the peptide that binds a protein of Plasmodium is
   a) X₄-NLQNCKKLRLLELGYNKI-X₅ (SEQ ID NO:7)
   b) X₆-YRKTIIHILPQLKQLD-X₇ (SEQ ID NO:8)
   c) or a proteolysis-resistant peptide deriving from SEQ ID NO: 7 or 8 by one or more chemical modifications, or a substantially homologous peptide deriving from SEQ ID NO:7 or 8 by one or more conservative substitutions
wherein X₄, X₅, X₆, X₇ are each independently vacant, or are an amino acid of sequence of one to 2 amino acids.

Another subject of the invention is a nucleic acid that encodes the chimeric peptide construct as defined herein. A further subject of the invention is a vector comprising said nucleic acid, which is preferably an adenovirus or a lentivirus vector.

The chimeric peptides, nucleic acid or vector are useful in preventing or treating malaria in a patient.

### Legends to the Figures:

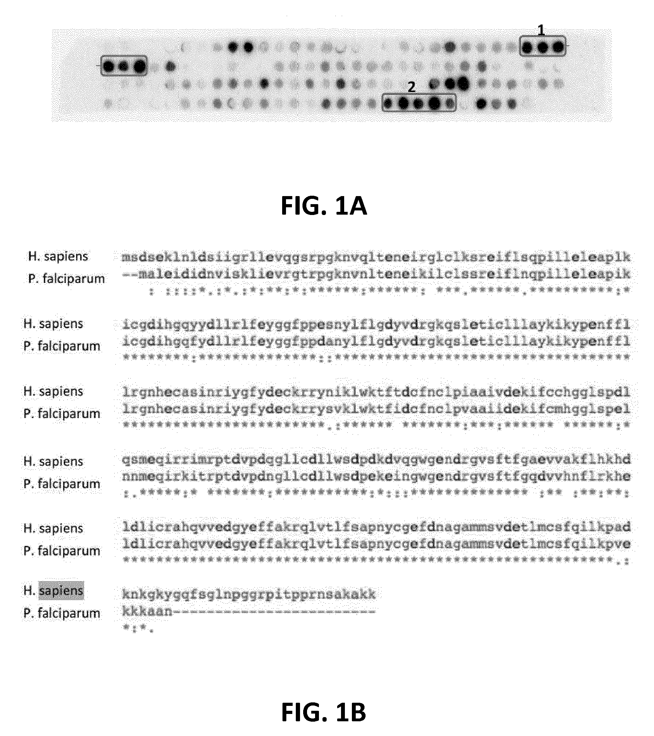

**Figure 1** shows determination of the binding site of PP1 to PfLRR1. A) Overlapping dodecapeptides with two amino acid shift covering the whole murine PfLRR1 protein were bound to a solid support. The membrane was incubated sequentially with PP1 protein, and anti- PP1 antibody, followed by a peroxidase-labeled secondary antibody. The membrane was revealed with ECL system. PfLRR1.1 and PfLRR1.2 are shown. B) PP1 sequence alignment between human and *P. falciparum* species (SEQ ID NO:16 and 15).

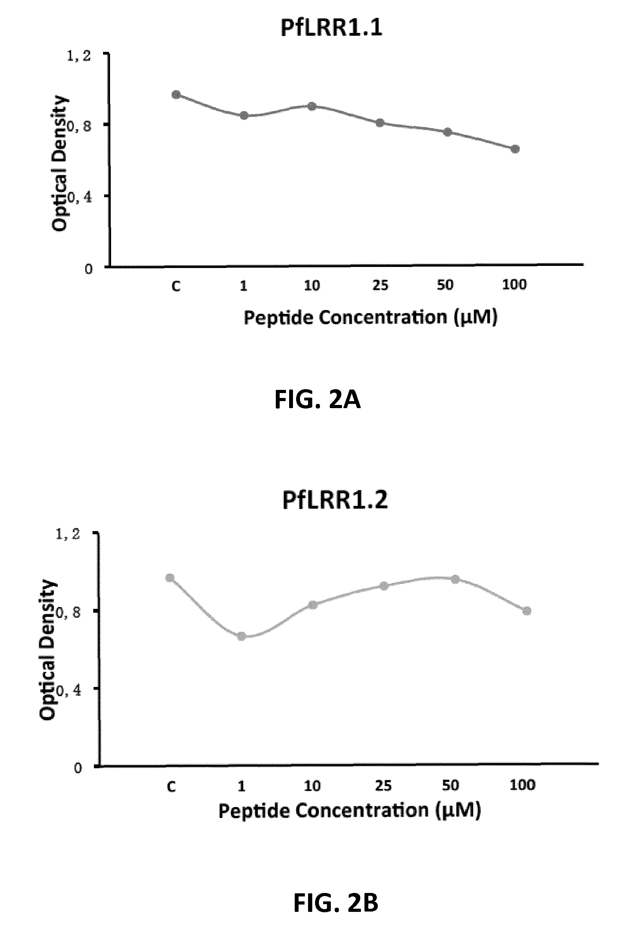

**Figures 2A and 2B** show in vitro cell viability assay. Primary hepatocytes were treated with or without various concentrations of PfLRR1.2 or PfLRR1.2 peptides. Absorbance was recorded 72h after. The results show that the peptides are not toxic for primary human hepatocytes.

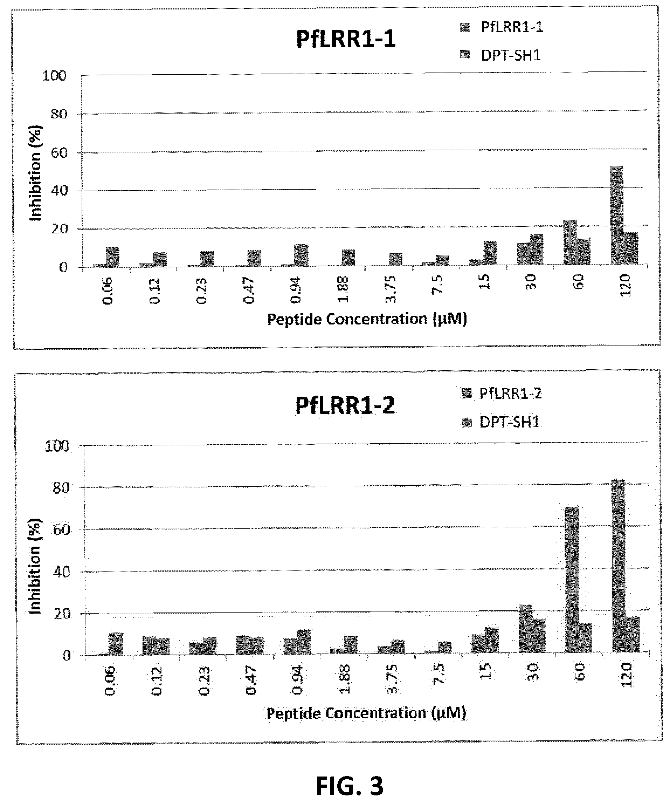

**Figure 3** shows in vitro effect of chimeric peptides on the erytrocytic stage of *P. falciparum.* The assay was done using parasitemia of 0.5% and hematocrit of 1%. The peptides were added at various concentrations and incubated for 48h and then, fixed. Samples were analyzed by flow cytometry. Results are expressed as percentage of growth inhibition.

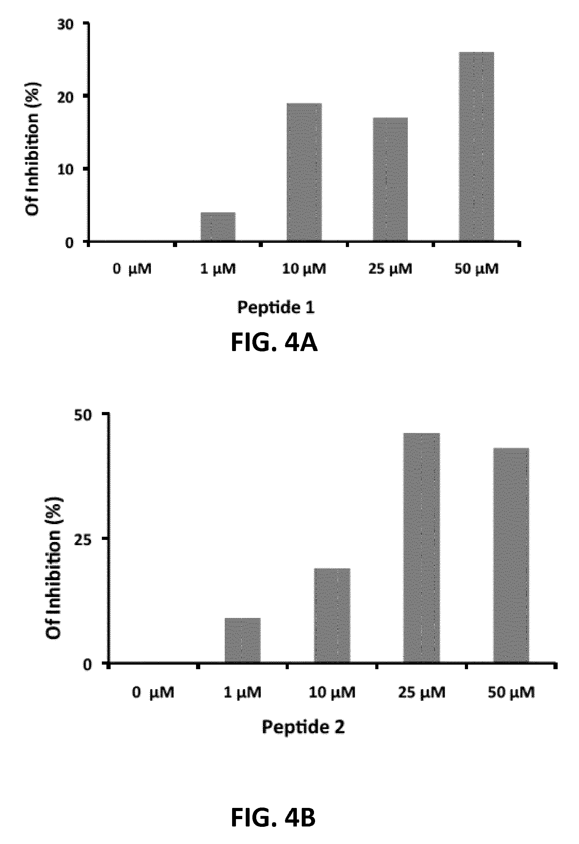

**Figures 4A and 4B** show in vitro effect of chimeric peptides PfLRR1.1 (peptide 1) and PfLRR1.2 (peptide 2) on the hepatic stage of the *P. falciparum.* Primary human hepatocytes were infected with *P. falciparum* sporozoites. Three hours after infection, the peptides were added to the culture medium. The peptides were added each 48h to complete 6 days of treatment. The culture was fixed with paraformaldehyde (PFA) and analysed by fluorescence microscopy.

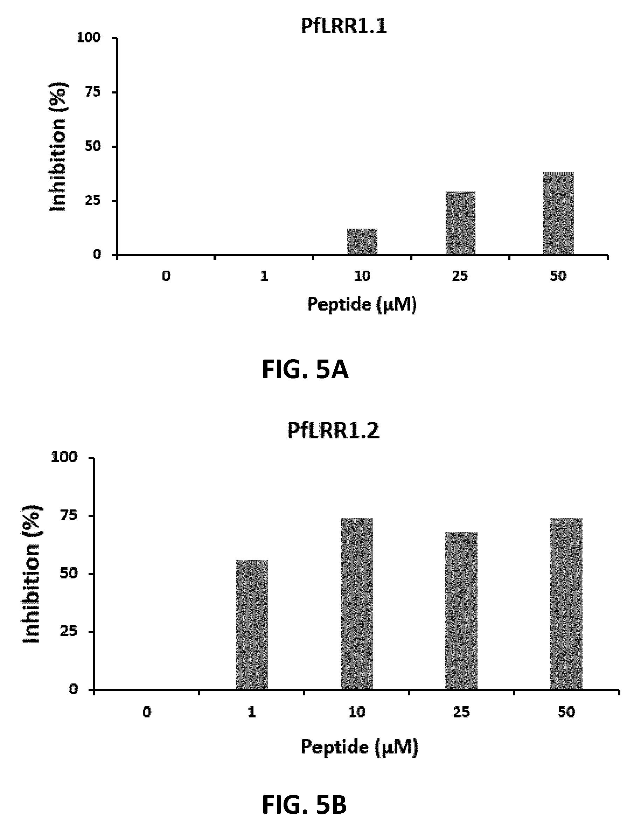

**Figures 5A and 5B** show in vitro effect of peptides PfLRR1.1 and PfLRR1.2 on the hepatic stage of the *P. vivax.* Primary human hepatocytes were infected with *P. vivax* sporozoites. Three hours after infection, the peptides were added to the culture medium at different concentrations. The peptides were added each 48h to complete 6 days of treatment. The culture was fixed with PFA and analysed by fluorescence microscopy.

### Detailed description of the invention:

### Definitions:

The term "patient" or "subject" refers to a human or a mammal in need of a treatment or prophylaxis against malaria. The "patient" or "subject" is typically a human subject. The subject can be male or female, a child or an adult. A subject can be one who has been previously diagnosed or identified as having malaria. Alternatively, a subject can also be one who has not been previously diagnosed as having malaria, but who is at risk of developing such condition, e.g. due to infection or due to travel within a region in which malaria is prevalent. For example, a subject may be infected with Plasmodium but asymptomatic or may show one or more symptoms for malaria. The subject may be infected with any Plasmodium species, in particular with Plasmodium falciparum or Plasmodium vivax. Symptoms appear usually one or two weeks after the infective mosquito bite. The first symptoms - fever, headache, chills and vomiting - may be mild and difficult to recognize as malaria. If not treated within 24 hours, P. falciparum malaria can progress to severe illness, often leading to death. The most severe manifestations are cerebral malaria, anemia and kidney, liver and lung dysfunctions.

As used herein, the term "treatment" or "therapy" includes curative and/or prophylactic treatment. More particularly, curative treatment refers to any of the alleviation, amelioration and/or elimination, reduction and/or stabilization (e.g., failure to progress to more advanced stages) of a symptom, as well as delay in progression of a symptom of a particular disorder.

Prophylactic treatment refers to any of: halting the onset, reducing the risk of development, reducing the incidence, delaying the onset, reducing the development, as well as increasing the time to onset of symptoms of a particular disorder. For instance, primary chemoprophylaxis regimens involve taking a medicine before travel, during travel, and for a period of time after leaving the malaria endemic area. Beginning the drug before travel allows the antimalarial agent to be in the blood before the traveler is exposed to malaria parasites. Alternatively, presumptive antirelapse therapy (also known as terminal prophylaxis) uses a medication towards the end of the exposure period (or immediately thereafter) to prevent relapses or delayed-onset clinical presentations of malaria caused by hypnozoites (dormant liver stages) of *P. vivax* or *P. ovale.*

The term "penetrating peptide" or "cell-penetrating peptide" (or "CPP") or "shuttle peptide", as used interchangeably, means that the peptide is able to translocate into cells without causing substantial membrane damage, and can be used as a vector of other molecules when linked to them. The terms refer to cationic cell penetrating peptides, also called transport peptides, carrier peptides, or peptide transduction domains. The CPP, as shown herein, have the capability of inducing cell penetration of a peptide fused to the CPP within 30%, 40%, 50%, 60%, 70%, 80%, 90%, or 100% of cells of a given cell culture population, including all integers in between, and allow macromolecular translocation within multiple tissues in vivo upon systemic administration. A cell-penetrating peptide may also refer to a peptide which, when brought into contact with a cell under appropriate conditions, passes from the external environment in the intracellular environment, including the cytoplasm, organelles such as mitochondria, or the nucleus of the cell, in conditions significantly greater than passive diffusion. This property may be assessed by various methods known by the skilled person. Cell-Penetrating Peptides (CPPs) are also known as protein transduction domains (PTDs), membrane translocating sequences (MTSs), or Trojan peptides.

Two amino acid sequences are "homologous", "substantially homologous" or "substantially similar" when one or more amino acid residue are replaced by a biologically similar residue or when greater than 80 % of the amino acids are identical, or greater than about 90 %, preferably greater than about 95%, are identical or similar (functionally identical). Preferably, the similar or homologous sequences are identified by alignment using, for example, the GCG (Genetics Computer Group, Program Manual for the GCG Package, *Version* 7, Madison, Wisconsin) pileup program, or any of the programs known in the art (BLAST, FASTA, etc.). Preferably, these homologous peptides do not include two cysteine residues, so that cyclization is prevented. Preferably the homologous sequences differ by mutations, such as substitutions, insertions and/or deletions of one or several amino acids. Preferably the homologous sequences differ only by conservative substitution(s).

The term "conservative substitution" as used herein denotes the replacement of an amino acid residue by another, without altering the overall conformation and function of the peptide, including, but not limited to, replacement of an amino acid with one having similar properties (such as, for example, polarity, hydrogen bonding potential, acidic, basic, shape, hydrophobic, aromatic, and the like). Amino acids with similar properties are well known in the art. For example, arginine, histidine and lysine are hydrophilic-basic amino acids and may be interchangeable. Similarly, isoleucine, a hydrophobic amino acid, may be replaced with leucine, methionine or valine. Neutral hydrophilic amino acids, which can be substituted for one another, include asparagine, glutamine, serine and threonine.

By "substituted" or "modified" the present invention includes those amino acids that have been altered or modified from naturally occurring amino acids.

As such, it should be understood that in the context of the present invention, a conservative substitution is recognized in the art as a substitution of one amino acid for another amino acid that has similar properties. Examples of conservative substitutions are set out in the Table 1 below:

**Table 1. Conservative Substitutions I**

| SIDE CHAIN CHARACTERISTIC | AMINO ACID |
|---|---|
| Non-polar | G A P I L V |
| Polar-uncharged | C S T M N Q |
| Polar-charged | D E K R |
| Aromatic | H F W Y |
| Other | N Q D E |

Alternatively, conservative amino acids can be grouped as described in Lehninger, 1975, as set out in Table 2, immediately below.

**Table 2. Conservative Substitutions II**

| SIDE CHAIN CHARACTERISTIC | AMINO ACID |
|---|---|
| Non-polar (hydrophobic) | |
| A. Alipliatic: | A L I V P |
| B. Aromatic: | F W |
| C. Sulfur-containing: | M |
| D. Borderline: | G |
| | |
| Uncharged-polar | |
| A. Hydroxyl: | S T Y |
| B. Amides: | N Q |
| C. Sulfhydryl: | C |
| D. Borderline: | G |
| Positively Charged (Basic): | K R H |
| Negatively Charged (Acidic): | D E |

As still another alternative, exemplary conservative substitutions are set out in Table 3, immediately below.

**Table 3. Conservative Substitutions III**

| Original Residue | Exemplary Substitution |
|---|---|
| Ala (A) | Val (V), Leu (L), lie (I) |
| Arg (R) | Lys (K), Gln (Q), Asn (N) |
| Asn (N) | Gln (Q), His (H), Lys (K), Arg (R) |
| Asp (D) | Glu (E) |
| Cys (C) | Ser (S) |
| Gln (Q) | Asn (N) |
| Glu (E) | Asp (D) |
| His (H) | Asn (N), Gin (Q), Lys (K), Arg (R) |
| Ile (I) | Leu (L), Val (V), Met (M), Ala (A), Phe (F) |
| Leu (L) | Ile (I), Val (V), Met (M), Ala (A), Phe (F) |
| Lys (K) | Arg (R), Gln (Q), Asn (N) |
| Met (M) | Leu (L), Phe (F), Ile (I) |
| Phe (F) | Leu (L), Val (V), Ile (I), Ala (A) |
| Pro (P) | Gly (G) |
| Ser (S) | Thr(T) |
| Thr (T) | Ser (S) |
| Trp (W) | Tyr (T) |
| Tyr (Y) | Trp (W), Phe (F), Thr (T), Ser (S) |
| Val (V) | Ile (I), Leu (L), Met (M), Phe (F), Ala (A) |

### Peptide that binds a protein of Plasmodium:

The invention makes use of a peptide that binds a protein of Plasmodium. Such peptide is a fragment of PfLRR1 protein, or derives therefrom. Advantageously, the peptide binds to PP1.

The present invention makes use of a peptide that binds a protein of Plasmodium that is
a) X₄-NLQNCKKLRLLELGYNKI-X₅ (SEQ ID NO:7)
b) X₆-YRKTIIHILPQLKQLD-X₇ (SEQ ID NO:8)
c) or a proteolysis-resistant peptide deriving from SEQ ID NO: 7 or 8 by one or more chemical modifications, or a substantially homologous peptide deriving from SEQ ID NO: 6 or 7 by one or more conservative substitutions
wherein X₄, X₅, X₆, X₇ are each independently vacant, or are an amino acid of sequence of one to 2 amino acids.

According to one embodiment, the peptide that binds a protein of Plasmodium binds PP1.

According to an embodiment, the peptide that binds a protein of Plasmodium is X₄-NLQNCKKLRLLELGYNKI-X₅, wherein X₄ is glutamic acid (E), IE, , and/or X₅ is R, or RM (SEQ ID NO:9).
In a preferred embodiment, the peptide that binds a protein of Plasmodium is IENLQNCKKLRLLELGYNKIRM (SEQ ID NO:10).

According to another embodiment, the peptide that binds a protein of Plasmodium is X₆-YRKTIIHILPQLKQLD-X₇ (SEQ ID NO:12), wherein X₆ is asparagine (N), EN and/or X₇ is alanine (A), or AL.
In a preferred embodiment, 9. The peptide that binds a protein of Plasmodium is ENYRKTIIHILPQLKQLDAL (SEQ ID NO:13).

### Cell Penetrating Peptides:

The peptide that binds a protein of Plasmodium is linked with at least one cell penetrating peptide, forming a chimeric peptide construct.

The peptide that binds a protein of Plasmodium is fused at the N-term or C-term of the penetrating peptide. Preferably, the cell penetrating peptide is fused at the N-terminus of the peptide that binds a protein of Plasmodium.

In a particular embodiment, the peptide that binds a protein of Plasmodium may be linked to two, three or more penetrating peptides.

The cell penetrating peptide is:
a) X₁-KKKIK-**Ψ**-EI-X₂-X₃(SEQ ID NO:1)
Wherein X₁ is vacant, is a lysine residue, or valine-lysine;
X₂ is vacant, is a lysine residue, or lysine-isoleucine;
X₃ is vacant or is an amino acid sequence of one to 4 amino acids;
and Ψ is any amino-acid;
or a proteolysis-resistant peptide deriving from SEQ ID NO:1 by one or more chemical modifications, or a substantially homologous peptide, especially peptides deriving from SEQ ID NO:1 by one or more conservative substitutions.

More preferably, cell-penetrating peptide is :
X₁-KKKIK-**Ψ**-EI-X₂-X₃(SEQ ID NO:2)
wherein Ψ is arginine, alanine, lysine, or asparagine,
and X₁ is valine-lysine;
X₂ is lysine-isoleucine;
and X₃ is vacant.

Advantageously, the cell-penetrating peptide is VKKKKIKREIKI (SEQ ID NO:3), VKKKKIKAEIKI (SEQ ID NO:4), VKKKKIKKEIKI (SEQ ID NO:5) or VKKKKIKNEIKI (SEQ ID NO:5). More preferably, cell-penetrating peptide is VKKKKIKAEIKI (SEQ ID NO:4).

### Chimeric constructs:

The chimeric peptide construct may preferably have a length comprised between 17 to 80 amino acids, preferably between 20 to 70 amino acids, still preferably between 23 to 40 amino acids.

In a preferred embodiment, the chimeric peptide construct is selected from the group consisting of:
VKKKKIKAEIKI- IENLQNCKKLRLLELGYNKIRM (SEQ ID NO:11) ;
VKKKKIKAEIKI- ENYRKTIIHILPQLKQLDAL (SEQ ID NO:14);
or homologous or proteolysis-resistant peptides deriving thereof.

### Peptide preparation:

Peptides described herein can be synthesized using standard synthetic methods known to those skilled in the art, for example chemical synthesis or genetic recombination. In a preferred embodiment, peptides are obtained by stepwise condensation of amino acid residues, either by condensation of a preformed fragment already containing an amino acid sequence in appropriate order, or by condensation of several fragments previously prepared, while protecting the amino acid functional groups except those involved in peptide bond during condensation. In particular, the peptides can be synthesized according to the method originally described by Merrifield.

Examples of chemical synthesis technologies are solid phase synthesis and liquid phase synthesis. As a solid phase synthesis, for example, the amino acid corresponding to the C-terminus of the peptide to be synthesized is bound to a support which is insoluble in organic solvents, and by alternate repetition of reactions, one wherein amino acids with their amino groups and side chain functional groups protected with appropriate protective groups are condensed one by one in order from the C-terminus to the N- terminus, and one where the amino acids bound to the resin or the protective group of the amino groups of the peptides are released, the peptide chain is thus extended in this manner. Solid phase synthesis methods are largely classified by the tBoc method and the Fmoc method, depending on the type of protective group used. Typically used protective groups include tBoc (t-butoxycarbonyl), CI-Z (2-chlorobenzyloxycarbonyl), Br-Z (2-bromobenzyloyycarbonyl), Bzl (benzyl), Fmoc (9-fluorenylmcthoxycarbonyl), Mbh (4, 4'-dimethoxydibenzhydryl), Mtr (4-methoxy-2, 3, 6-trimethylbenzenesulphonyl), Trt (trityl), Tos (tosyl), Z (benzyloxycarbonyl) and Clz-Bzl (2, 6-dichlorobenzyl) for the amino groups; NO2 (nitro) and Pmc (2,2, 5,7, 8-pentamethylchromane-6-sulphonyl) for the guanidino groups); and tBu (t-butyl) for the hydroxyl groups). After synthesis of the desired peptide, it is subjected to the de-protection reaction and cut out from the solid support. Such peptide cutting reaction may be carried with hydrogen fluoride or tri-fluoromethane sulfonic acid for the Boc method, and with TFA for the Fmoc method.

Alternatively, the peptide may be synthesized using recombinant techniques. In this case, a nucleic acid and/or a genetic construct comprising or consisting of a nucleotidic sequence encoding a peptide according to the invention, polynucleotides with nucleotidic sequences complementary to one of the above sequences and sequences hybridizing to said polynucleotides under stringent conditions.

The invention further relates to a genetic construct consisting of or comprising a polynucleotide as defined herein, and regulatory sequences (such as a suitable promoter(s), enhancer(s), terminator(s), etc.) allowing the expression (*e.g.* transcription and translation) of a peptide according to the invention in a host cell.

Thus, in another aspect, the invention relates to a host or host cell that expresses (or that under suitable circumstances is capable of expressing) a peptide of the invention; and/or that contains a polynucleotide of the invention or genetic construct of the invention.

The method of producing the peptide may optionally comprise the steps of purifying said peptide, chemically modifying said peptide, and/or formulating said peptide into a pharmaceutical composition.

### Further protection against proteolysis:

The N- and C-termini of the peptides described herein may be optionally protected against proteolysis. For instance, the N-terminus may be in the form of an acetyl group, and/or the C-terminus may be in the form of an amide group. Internal modifications of the peptides to be resistant to proteolysis are also envisioned, e.g. wherein at least a -CONH- peptide bond is modified and replaced by a (CH2NH) reduced bond, a (NHCO) retro-inverso bond, a (CH2-O) methylene-oxy bond, a (CH2-S) thiomethylene bond, a (CH2CH2) carba bond, a (CO-CH2) cetomethylene bond, a (CHOH-CH2) hydroxyethylene bond), a (N-N) bound, a E-alcene bond or also a -CH=CH-bond.

For instance the peptide may be modified by acetylation, acylation, amidation, crosslinking, cyclization, disulfide bond formation, formation of covalent cross-links, formation of cysteine, formation of pyroglutamate, formylation, gamma-carboxylation, glycosylation, GPI anchor formation, hydroxylation, iodination, methylation, myristylation, oxidation, phosphorylation, and the like.

The peptides of the invention may be composed of amino acid(s) in D configuration, which render the peptides resistant to proteolysis. They may also be stabilized by intramolecular crosslinking, e.g. by modifying at least two amino acid residues with olefinic side chains, preferably C3-C8 alkenyl chains, preferably penten-2-yl chains) followed by chemical crosslinking of the chains, according to the so-called "staple" technology described in Walensky et al, 2004. For instance, amino acids at position i and i+4 to i+7 can be substituted by non-natural aminoacids that show reactive olefinic residues. All these proteolysis-resistant chemically-modified peptides are encompassed in the present invention.

In another aspect of the invention, peptides are covalently bound to a polyethylene glycol (PEG) molecule by their C-terminal terminus or a lysine residue, notably a PEG of 1500 or 4000 MW, for a decrease in urinary clearance and in therapeutic doses used and for an increase of the half-life in blood plasma. In yet another embodiment, peptide half-life is increased by including the peptide in a biodegradable and biocompatible polymer material for drug delivery system forming microspheres. Polymers and copolymers are, for instance, poly(D,L-lactide-co-glycolide) (PLGA) (as illustrated in US2007/0184015, SoonKap Hahn et al).

### Nucleic acids:

The invention also relates to a polynucleotide comprising or consisting of a nucleotide sequence encoding a chimeric peptide construct according to the invention.

The invention further relates to a genetic construct consisting of or comprising a polynucleotide as defined herein, and regulatory sequences (such as a suitable promoter(s), enhancer(s), terminator(s), etc.) allowing the expression (e.g. transcription and translation) of a peptide according to the invention in a host cell.

The genetic constructs of the invention may be DNA or RNA, preferably cDNA, and are preferably double-stranded DNA. The genetic constructs of the invention may also be in a form suitable for transformation of the intended host cell or host organism, in a form suitable for integration into the genomic DNA of the intended host cell or in a form suitable for independent replication, maintenance and/or inheritance in the intended host organism. For instance, the genetic constructs of the invention may be in the form of a vector, such as for example a plasmid, cosmid, YAC, a viral vector or transposon. In particular, the vector may be an expression vector, i.e. a vector that can provide for expression in vitro and/or in vivo (e.g. in a suitable host cell, host organism and/or expression system).

In a preferred but non-limiting aspect, a genetic construct of the invention comprises i) at least one nucleic acid of the invention; operably connected to ii) one or more regulatory elements, such as a promoter and optionally a suitable terminator; and optionally also iii) one or more further elements of genetic constructs such as 3'- or 5'-UTR sequences, leader sequences, selection markers, expression markers/reporter genes, and/or elements that may facilitate or increase (the efficiency of) transformation or integration.

In a particular embodiment, the nucleic acid encoding the cell-penetrating peptide is coupled or fused to a nucleic acid that encodes a peptide that binds a protein of Plasmodium as described herein.

The nucleic acid may especially be carried by a viral vector, such as an adenovirus or a lentivirus, for *ex vivo* or *in vivo* infection and expression of the chimeric peptide construct according to the invention.

### Medical use of the peptides of the invention:

The inventors have shown that the chimeric peptides of the invention can be useful in the preparation of a drug for prevention or treatment of malaria. According to the inventors, the chimeric peptides are able to disrupt plasmodial PP1-PfLRR1 protein-protein interactions by acting as highly specific competitors of this interaction, thus inhibiting specific cellular processes which are dependent upon this discrete protein-protein interaction. This approach was successful not only against *P. falciparum* but also *P. vivax.* Therefore, there is reason to believe that the PP1-PfLRR1 proteins are conserved across the genus Plasmodium and that the chimeric peptide possess an activity against subspecies *P. falciparum, P. vivax, P. malariae, P. ovale and P. knowlesi.* Moreover, inventors believe that resistance to chimeric peptides of the invention is unlikely as mutations of both protein partners would be necessary for effective resistance. Furthermore, the inventors have shown that the chimeric peptides show no toxicity on tissues such as hepatocytes.

The inventors have also shown that the chimeric peptide can be useful for prevention or treatment of blood and liver stages of malaria, especially malaria caused by *P. falciparum* or *P. vivax* infection.

The chimeric peptides as defined herein, or nucleic acids that encode said peptides, are useful in treatment or prophylaxis of malaria. In preferred embodiments malaria is caused by *P. falciparum, P. vivax, P.ovale, P. malariae or P. knowlesi.*

More particularly the peptides described herein (or nucleic acids that encode said peptides) are useful in the treatment or prophylaxis of malaria caused by a plasmodium which exhibit a resistance to antimalarial drugs such as chloroquine, artemisinin, artemisinin derivatives or artemisinin-based combination therapy (ACT).

The antimalarial therapy of the invention is helpful in eradicating any hypnozoite, and/or preventing or delaying relapses.

The peptides (or nucleic acids that encode said peptides) described herein may be administered as separate tablets, capsules, solution for injection or any other pharmaceutically acceptable formulation together with one or more separate or combined antimalarial drugs.

The peptides (or nucleic acids that encode said peptides) described herein may also be administered as a combination formulation with one or more of said antimalarial drugs.

It is thus described a method of treatment or prophylaxis of malaria in a patient in need thereof, which method comprises administering said patient with a chimeric peptide construct of the invention, or a nucleic acid encoding said construct, optionally in combination with antimalarial drug.

### Pharmaceutical compositions and routes of administration:

The peptides (or nucleic acid that encodes said peptide) may be formulated in association with a pharmaceutically acceptable carrier.

The pharmaceutical composition may also include any other active principle, such as in particular an antimalarial agent.

Many routes of administration are known in the art and depending on the condition of the patient, oral, transdermal, or subcutaneous routes of administration may be relevant.

The preparation of a pharmacological composition that contains active ingredients dissolved or dispersed therein is well understood in the art and need not be limited based on formulation. Typically such compositions are prepared as injectables either as liquid solutions or suspensions; however, solid forms suitable for solution, or suspensions, in liquid prior to use can also be prepared. The preparation can also be emulsified. In particular, the pharmaceutical compositions may be formulated in solid dosage form, for example capsules, tablets, pills, powders, dragees or granules.

The choice of vehicle and the content of active substance in the vehicle are generally determined in accordance with the solubility and chemical properties of the active compound, the particular mode of administration and the provisions to be observed in pharmaceutical practice. For example, excipients such as lactose, sodium citrate, calcium carbonate, dicalcium phosphate and disintegrating agents such as starch, alginic acids and certain complex silicates combined with lubricants such as magnesium stearate, sodium lauryl sulphate and talc may be used for preparing tablets. To prepare a capsule, it is advantageous to use lactose and high molecular weight polyethylene glycols. When aqueous suspensions are used they can contain emulsifying agents or agents which facilitate suspension. Diluents such as sucrose, ethanol, polyethylene glycol, propylene glycol, glycerol and chloroform or mixtures thereof may also be used.

Preparation can involve the formulation of the desired molecule with an agent, such as injectable microspheres, bio-erodible particles, polymeric compounds (such as polylactic acid or polyglycolic acid), beads or liposomes, that may provide controlled or sustained release of the product.

The dosing is selected by the skilled person so that a prophylactic or treatment against malaria effect is achieved, and depends on the route of administration and the dosage form that is used. Total daily dose of peptides (or nucleic acid that encodes said peptide) administered to a subject in single or divided doses may be in amounts, for example, of from about 0.001 to about 50 mg/kg body weight daily and preferably 0.01 to 10 mg/kg/day. A daily dosage of about 5mg/kg is preferred. Dosage unit compositions may contain such amounts of such submultiples thereof as may be used to make up the daily dose. It will be understood, however, that the specific dose level for any particular patient will depend upon a variety of factors including the body weight, general health, sex, diet, time and route of administration, rates of absorption and excretion, combination with other drugs and the severity of the particular disease being treated.

Preferably the peptide construct (or nucleic acid that encodes said peptide) is administered once a day during a period of at least one week, preferably at least two weeks.

Further aspects and advantages of the present invention will be disclosed in the following experimental section, which should be regarded as illustrative and not limiting the scope of the present application.

### EXAMPLES:

### Example 1: Identification of binding sites of PfLRR1 to PP1

### 1.1. Materials and methods

### Peptide synthesis and sequence

Peptides were synthesized in an automated multiple peptide synthesizer with solid phase procedure and standard Fmoc chemistry. The purity and composition of the peptides were confirmed by reverse phase HPLC and by amino acid analysis. These peptides were used for protein-protein interaction competition studies or cell culture.

Overlapping dodecapeptides scanning the whole LRR1 sequence were prepared by automated spot synthesis (Abimed, Langerfeld, Germany) onto an amino-derived cellulose membrane, as described (Frank and Overwing, 1996, Gausepohl et al., 1992). The membrane was saturated using 3% non-fat dry milk/3% BSA, incubated with purified PP1 protein and after several washing steps, incubated with anti-PP1 antibody, followed by PO-conjugated secondary antibody. Positive spots were visualized using the ECL system.

### 1.2. Results

### Identification of binding site of PfLRR1 to PP1

To identify peptides containing LRR1 sequence able to in vitro bind to PP1, the whole sequence of LRR1 was synthetized as series of dodecapeptides that were bound to a nitrocellulose support.

The inventors identified two overlapping sequences, one of six dodecapeptides and the other one of five dodecapeptides (Figure 1A). These two sequences are IENLQNCKKLRLLELGYNKIRM (SEQ ID NO:10) and ENYRKTIIHILPQLKQLDAL (SEQ ID NO:13).

### Example 2: Design of chimeric peptides PfLRR1.1 and PfLRR1.2

### Peptide synthesis

The inventors chemically synthesized two cell penetrating peptides composed of a shuttle, DPT-Sh1 (VKKKKIKAEIKI, SEQ ID NO:4) associated to the binding sites of PfLRR1 to PP1. The chimeric peptides are
PfLRR1.1 : VKKKKIKAEIKI- ENYRKTIIHILPQLKQLDAL (SEQ ID NO:14).
PfLRR1.2 : VKKKKIKAEIKI- IENLQNCKKLRLLELGYNKIRM (SEQ ID NO:11).

### Example 3: In vitro effect of PfLRR1.1 and PfLRR1.2 peptides on of P. falciparum growth in erythrocytic stage.

### 3.1 Material and methods

The growth inhibition assay was described in detail in Fréville et al. (2013). Briefly, assays were carried out in 96-well plates with a starting parasitemia of 0.5% at a haematocrit of 1% using SYBR Green I (Kelly et al., 2009). The peptides were added to the culture at different concentrations ranging from 100 µM to 48 nM (final concentrations) in a volume of 250 µl of RPMI-AlbuMAX (0.5%) and incubated for an additional 48 h to allow all stages to complete one cycle. Cultures were stained for 30 minutes in the dark with SYBR Green I 1X (Invitrogen) diluted in 20 mM Tris pH8.8, 138 mM NaCl, and fixed with 1% paraformaldehyde. Fixed parasited red blood cells (RBC) were stored at 4°C in the dark until flow cytometry analysis. Parasite growth was assessed by flow cytometry on a BD FACSCantoll (BD Biosciences). Cell pairs were excluded from the analysis using a forward scatter (FSC)-width versus FSC-area dot plot. Infected and uninfected erythrocytes were gated on the basis of their FSC and side scatter (SSC) signals. Fluorescence analysis (Green fluorescence) was performed using BD FACSDiva software (version 6.1.3, BD Biosciences) on a total of 200,000 acquired events. Fluorescence was observed as described by Izumiyama et al. (2009) on a two-parameter dot plot (Green fluorescence-FSC). Fluorescence of noninfected RBC was adjusted to plot between 100 and 102. Results are expressed as the percentage of growth inhibition.

### 3.2 Results

The inventors analyzed the capacity of PfLRR1.1 and PfLRR1.2 peptides to inhibit erythrocytic stage of P. *falciparum* in vitro, in comparison with the control Mut3DPTSh1 shuttle peptide (VKKKKIKAEIKI, SEQ ID NO:4). As illustrated on Figure 3, DPT-Sh1 did not show any activity against the parasite whereas increasing concentrations of both PfLRR1-1 and PfLRR1-2 exhibited significant inhibitory activity on blood stages of *P. falciparum.* The assay was reproduced twice and percentages of inhibition are shown on Figure 3.

### Example 4: In vitro effect of PfLRR1.1 and PfLRR1.2 peptides on P. falciparum and P. vivax growth in hepatic stage.

### 4.1 Material and methods

### Parasites

P. falciparum sporozoites were obtained from infected salivary glands of Anopheles stephensi. Infected salivary glands were removed by hand dissection and crushed in a potter allowing sporozoites extraction. The parasites were recovered after filtration and 2 min centrifugation at 15,000g. Primary hepatocytes Human hepatocytes were isolated from liver segments obtained from adult patients undergoing partial hepatectomy. Simian hepatocytes were isolated from liver segments from healthy M. fascicularis. All hepatocytes were isolated as previously described (Silvie et al., 2003).

### Primary hepatocytes

Human hepatocytes were isolated from liver segments obtained from adult patients undergoing partial hepatectomy. Simian hepatocytes were isolated from liver segments from healthy M. fascicularis. All hepatocytes were isolated as previously described (Silvie et al.,2003).

### Hepatocyte infection and peptide assay

1x10⁵ P. falciparum sporozoites resuspended in complete medium were added to the host cell culture (human hepatocytes). The procedure of infection was previously described (Dembele et al., 2014). For peptide assay, the cultures were treated on the days indicated by adding the peptide to the medium. Parasite quantification Fluorescent parasites were detected by immunofluorescence. Following fixation, the culture plates were stained with anti-P. falciparum hsp70 polyclonal antibody followed by a fluorochrome-labelled secondary antibody. Parasites were detected under fluorescence microscope.

### Parasite quantification

Fluorescent parasites were detected by immunofluorescence. Following fixation, the culture plates were stained with anti-P. falciparum hsp70 polyclonal antibody followed by a fluorochrome-labelled secondary antibody. Parasites were detected under fluorescence microscope.

### Cell viability assays

The cell viability was determined using a colorimetric assay based on 3-(4,5-dimethylthiazol- 2-yl)-2,5 diphenyltetrazolium bromide (MTT). Cells were seeded at appropriate concentration in 96-well plates at day 0 and peptide was added to the medium and cell viability tested by MTT assay 72h after treatment following the manufacture's protocol. Results are expressed as relative percentages of metabolically active cells compared with untreated controls.

### 4.2 Results

The inventors analysed the capacity of PfLRR1.1 and PfLRR1.2 to inhibit P. falciparum growth in the hepatic stage. Human primary hepatocytes were infected with P. falciparum and 3h after infection, different concentrations of peptide PfLRR1.1 and 1.2 were added to the culture medium and then, the inhibition of parasite development was analysed. As illustrated on Figure 3, PfLRR1.1 and PfLRR1.2 show significant inhibitory activity on P. falciparum development, PfLRR1.2 being more active.

The effect of the peptides was also analysed on hepatic stage of P.vivax. Figures 5A and 5B show that both peptides have inhibitory effect on P. Vivax. As in the previous figure, the PfLRR1.2 peptide shows greater effect on plasmodium growth. The chimeric peptides of the invention possess an activity against *P. falciparum* at blood stage but also against hepatocyte stage. Interestingly, the peptides are also active against *P. vivax* hepatic stage.

### Example 5: Evaluation of the toxicity of the PfLRR1.1 and PfLRR1.2 peptide

The evaluation of the toxicity of PfLRR1.1 and PfLRR1.2 peptides was analysed on primary human hepatocytes at different concentrations. As shown in Figures 2A and 2B, the PfLRR1.1 and PfLRR1.2 peptides are not toxic for the host cell (primary human hepatocyte).

### REFERENCES

- Baird et al. Clinical Microbiology Reviews p. 36-57 January 2013 Volume 26 Number 1
- Cui et al. Am. J. Trop. Med. Hyg., 93(Suppl 3), 2015, pp. 57-68
- Daher et al., 2006, Molecular Microbiology, 60(3), 579-590
- Dembélé et al. Nat Med. 2014 Mar;20(3):307-12
- Fréville et al. J. BMC Biol. 2013 Jul 9;11:80.
- Frank, R., H. Overwin. 1996. Methods Mol. Biol. 66: 149-144;
- Gausepohl, et al. 1992. Pept. Res. 5: 315-314 ;
- Hollin et al., 2016 BMC Genomics Mar 17; 17:246.
- Izumiyama et al. Exp Parasitol. 2009 Feb;121(2):144-50
- Kelly et al. Malar J. 2009 Jan 23;8:19
- Pierrot et al., 2012, Current Pharmaceutical Design, 18, 3522-3530
- Silvie et al. D. Nat Med. 2003 Jan;9(1):93-6

## Claims

1. A chimeric peptide construct comprising a cell penetrating peptide linked to a peptide that binds a protein of Plasmodium,
wherein said cell-penetrating peptide is X₁-KKKIK-**Ψ**-EI-X₂-X3 (SEQ ID NO:1)
wherein X₁ is vacant, is a lysine residue, or valine-lysine;
X₂ is vacant, is a lysine residue, or lysine-isoleucine;
X₃ is vacant or is an amino acid sequence of one to 4 amino acids;
and Ψ is any amino-acid;
or a proteolysis-resistant peptide deriving from SEQ ID NO: 1 by one or more chemical modifications, or a substantially homologous peptide deriving from SEQ ID NO:1 by one or more conservative substitutions;
and the peptide that binds a protein of Plasmodium is
a) X₄-NLQNCKKLRLLELGYNKI-X₅ (SEQ ID NO:7)
b) X₆-YRKTIIHILPQLKQLD-X₇ (SEQ ID NO:8)
c) or a proteolysis-resistant peptide deriving from SEQ ID NO: 7 or 8 by one or more chemical modifications, or a substantially homologous peptide deriving from SEQ ID NO:7 or 8 by one or more conservative substitutions
wherein X₄, X₅, X₆, X₇ are each independently vacant, or are an amino acid of sequence of one to 2 amino acids.

2. The chimeric peptide construct of claim 1, wherein said cell-penetrating peptide is X₁-KKKIK-**Ψ**-EI-X₂-X₃(SEQ ID NO:2)
wherein Ψ is arginine, alanine, lysine, or asparagine.
and X₁ is valine-lysine;
X₂ is lysine-isoleucine;
and X₃ is vacant.

3. The chimeric peptide construct according to claim 2, wherein said cell-penetrating peptide is VKKKKIKREIKI (SEQ ID NO:3), VKKKKIKAEIKI (SEQ ID NO:4), VKKKKIKKEIKI (SEQ ID NO:5) or VKKKKIKNEIKI (SEQ ID NO:6).

4. The chimeric peptide construct according to any of claims 1 to 3, wherein the cell penetrating peptide is fused at the N-terminus of the peptide that binds a protein of Plasmodium.

5. The chimeric peptide construct according to any of claims 1 to 4, wherein said peptide that binds a protein of Plasmodium is
X₄-NLQNCKKLRLLELGYNKI-X₅ (SEQ ID NO:9), wherein X₄ is glutamic acid (E),
IE (isoleucine-glutamic acid), and/or X₅ is R, or RM.

6. The chimeric peptide construct according to claim 5, wherein said peptide that binds a protein of Plasmodium is IENLQNCKKLRLLELGYNKIRM (SEQ ID NO:10).

7. The chimeric peptide construct according to claim 6, wherein said chimeric peptide consists of peptide VKKKKIKAEIKI- IENLQNCKKLRLLELGYNKIRM (SEQ ID NO:11).

8. The chimeric peptide construct according to any of claims 1 to 4, wherein said peptide that binds a protein of Plasmodium is
X₆-YRKTIIHILPQLKQLD-X₇ (SEQ ID NO:12) wherein X₆ is asparagine (N), EN
(glutamic acid-asparagine) and/or X₇ is alanine (A), or AL (Alanine-Leucine).

9. The chimeric peptide construct according to claim 8, wherein said peptide that binds a protein of Plasmodium is ENYRKTIIHILPQLKQLDAL (SEQ ID NO:13).

10. The chimeric peptide construct according to claim 9, wherein said chimeric peptide consists of peptide VKKKKIKAEIKI-ENYRKTIIHILPQLKQLDAL (SEQ ID NO:14).

11. The chimeric peptide construct of any of claims 1 to 10, for use in preventing or treating malaria in a patient, preferably wherein the patient is infected with Plasmodium falciparum or Plasmodium vivax.

12. A nucleic acid that encodes the chimeric peptide construct as defined in any of claims 1 to 10.

13. A vector comprising the nucleic acid of claim 12, which is preferably an adenovirus or a lentivirus vector.

14. The nucleic acid of claim 12 or the vector of claim 13, for use in preventing or treating malaria in a patient.

15. Pharmaceutical composition comprising at least one peptide according to any one of claims 1 to 10, and a pharmaceutically acceptable vehicle and/or excipient.


SEQUENCE LISTING

<110>  UNIVERSITE PIERRE ET MARIE CURIE (Paris 6) et al

<120>  Chimeric peptides useful in malaria prophylaxis or treatment.

<130>  B2283EP

<160>  16

<170>  PatentIn version 3.5

<210>  1
<211>  11
<212>  PRT
<213>  Artificial Sequence

<220>
<223>  peptide

<220>
<221>  MISC_FEATURE
<222>  (1)..(1)
<223>  X is vacant, is a lysine residue, or valine-lysine

<220>
<221>  MISC_FEATURE
<222>  (7)..(7)
<223>  X is any amino acid

<220>
<221>  MISC_FEATURE
<222>  (10)..(10)
<223>  X is vacant, is lysine residue, or lysine-isoleucine

<220>
<221>  MISC_FEATURE
<222>  (11)..(11)
<223>  X is vacant or is an amino acid sequence of one to 4 amino acids

<400>  1

Xaa Lys Lys Lys Ile Lys Xaa Glu Ile Xaa Xaa
1               5                   10

<210>  2
<211>  11
<212>  PRT
<213>  Artificial Sequence

<220>
<223>  peptide

<220>
<221>  MISC_FEATURE
<222>  (1)..(1)
<223>  X is valine-lysine

<220>
<221>  MISC_FEATURE

<222>  (7)..(7)
<223>  X is arginine, alanine, lysine, or asparagine

<220>
<221>  MISC_FEATURE
<222>  (10)..(10)
<223>  X is lysine-isoleucine

<220>
<221>  MISC_FEATURE
<222>  (10)..(10)
<223>  X is vacant

<220>
<221>  MISC_FEATURE
<222>  (11)..(11)
<223>  X is vacant

<400>  2

Xaa Lys Lys Lys Ile Lys Xaa Glu Ile Xaa Xaa
1               5                   10


<210>  3
<211>  12
<212>  PRT
<213>  Artificial Sequence

<220>
<223>  peptide

<400>  3

Val Lys Lys Lys Lys Ile Lys Arg Glu Ile Lys Ile
1               5                   10


<210>  4
<211>  12
<212>  PRT
<213>  Artificial Sequence

<220>
<223>  peptide

<400>  4

Val Lys Lys Lys Lys Ile Lys Ala Glu Ile Lys Ile
1               5                   10


<210>  5
<211>  12
<212>  PRT
<213>  Artificial Sequence

<220>
<223>  peptide

<400>  5

Val Lys Lys Lys Lys Ile Lys Lys Glu Ile Lys Ile

1               5                   10


<210>  6
<211>  12
<212>  PRT
<213>  Artificial Sequence

<220>
<223>  peptide

<400>  6

Val Lys Lys Lys Lys Ile Lys Asn Glu Ile Lys Ile
1               5                   10


<210>  7
<211>  20
<212>  PRT
<213>  Artificial Sequence

<220>
<223>  peptide


<220>
<221>  misc_feature
<222>  (1)..(1)
<223>  X is vacant, or an amino acid of sequence of one to 2 amino acids

<220>
<221>  misc_feature
<222>  (20)..(20)
<223>  X is vacant, or an amino acid of sequence of one to 2 amino acids

<400>  7

Xaa Asn Leu Gln Asn Cys Lys Lys Leu Arg Leu Leu Glu Leu Gly Tyr
1               5                   10                  15


Asn Lys Ile Xaa
            20


<210>  8
<211>  18
<212>  PRT
<213>  Artificial Sequence

<220>
<223>  peptide


<220>
<221>  misc_feature
<222>  (1)..(1)
<223>  X is vacant, or is an amino acid of sequence of one to 2 amino
       acids.

<220>
<221>  misc_feature

<222>  (18)..(18)
<223>  X is vacant, or is an amino acid of sequence of one to 2 amino
       acids.

<400>  8

Xaa Tyr Arg Lys Thr Ile Ile His Ile Leu Pro Gln Leu Lys Gln Leu
1               5                   10                  15

Asp Xaa

<210>  9
<211>  20
<212>  PRT
<213>  Artificial Sequence

<220>
<223>  peptide

<220>
<221>  MISC_FEATURE
<222>  (1)..(1)
<223>  X is glutamic acid (E) or IE (isoleucine-glutamic acid)

<220>
<221>  MISC_FEATURE
<222>  (20)..(20)
<223>  X is R, or RM

<400>  9

Xaa Asn Leu Gln Asn Cys Lys Lys Leu Arg Leu Leu Glu Leu Gly Tyr
1               5                   10                  15

Asn Lys Ile Xaa
            20

<210>  10
<211>  22
<212>  PRT
<213>  Artificial Sequence

<220>
<223>  peptide

<400>  10

Ile Glu Asn Leu Gln Asn Cys Lys Lys Leu Arg Leu Leu Glu Leu Gly
1               5                   10                  15

Tyr Asn Lys Ile Arg Met
            20

<210>  11
<211>  34

<212>  PRT
<213>  Artificial Sequence

<220>
<223>  peptide

<400>  11

Val Lys Lys Lys Lys Ile Lys Ala Glu Ile Lys Ile Ile Glu Asn Leu
1               5                   10                  15

Gln Asn Cys Lys Lys Leu Arg Leu Leu Glu Leu Gly Tyr Asn Lys Ile
            20                  25                  30

Arg Met

<210>  12
<211>  18
<212>  PRT
<213>  Artificial Sequence

<220>
<223>  peptide

<220>
<221>  MISC_FEATURE
<222>  (1)..(1)
<223>  X is asparagine (N), or EN (glutamic acid-asparagine)

<220>
<221>  MISC_FEATURE
<222>  (18)..(18)
<223>  X7 is alanine (A), or AL (Alanine-Leucine).

<400>  12

Xaa Tyr Arg Lys Thr Ile Ile His Ile Leu Pro Gln Leu Lys Gln Leu
1               5                   10                  15

Asp Xaa

<210>  13
<211>  20
<212>  PRT
<213>  Artificial Sequence

<220>
<223>  peptide

<400>  13

Glu Asn Tyr Arg Lys Thr Ile Ile His Ile Leu Pro Gln Leu Lys Gln
1               5                   10                  15

Leu Asp Ala Leu
            20

<210>  14
<211>  32
<212>  PRT
<213>  Artificial Sequence

<220>
<223>  peptide

<400>  14

Val Lys Lys Lys Lys Ile Lys Ala Glu Ile Lys Ile Glu Asn Tyr Arg
1               5                   10                  15

Lys Thr Ile Ile His Ile Leu Pro Gln Leu Lys Gln Leu Asp Ala Leu
            20                  25                  30

<210>  15
<211>  304
<212>  PRT
<213>  Plasmodium falciparum

<400>  15

Met Ala Leu Glu Ile Asp Ile Asp Asn Val Ile Ser Lys Leu Ile Glu
1               5                   10                  15

Val Arg Gly Thr Arg Pro Gly Lys Asn Val Asn Leu Thr Glu Asn Glu
            20                  25                  30

Ile Lys Ile Leu Cys Leu Ser Ser Arg Glu Ile Phe Leu Asn Gln Pro
        35                  40                  45

Ile Leu Leu Glu Leu Glu Ala Pro Ile Lys Ile Cys Gly Asp Ile His
    50                  55                  60

Gly Gln Phe Tyr Asp Leu Leu Arg Leu Phe Glu Tyr Gly Gly Phe Pro
65                  70                  75                  80

Pro Asp Ala Asn Tyr Leu Phe Leu Gly Asp Tyr Val Asp Arg Gly Lys
                85                  90                  95

Gln Ser Leu Glu Thr Ile Cys Leu Leu Leu Ala Tyr Lys Ile Lys Tyr
            100                 105                 110

Pro Glu Asn Phe Phe Leu Leu Arg Gly Asn His Glu Cys Ala Ser Ile
        115                 120                 125

Asn Arg Ile Tyr Gly Phe Tyr Asp Glu Cys Lys Arg Arg Tyr Ser Val
    130                 135                 140

Lys Leu Trp Lys Thr Phe Ile Asp Cys Phe Asn Cys Leu Pro Val Ala
145                 150                 155                 160

Ala Ile Ile Asp Glu Lys Ile Phe Cys Met His Gly Gly Leu Ser Pro
                165                 170                 175

Glu Leu Asn Asn Met Glu Gln Ile Arg Lys Ile Thr Arg Pro Thr Asp
            180                 185                 190

Val Pro Asp Asn Gly Leu Leu Cys Asp Leu Leu Trp Ser Asp Pro Glu
        195                 200                 205

Lys Glu Ile Asn Gly Trp Gly Glu Asn Asp Arg Gly Val Ser Phe Thr
    210                 215                 220

Phe Gly Gln Asp Val Val His Asn Phe Leu Arg Lys His Glu Leu Asp
225                 230                 235                 240

Leu Ile Cys Arg Ala His Gln Val Val Glu Asp Gly Tyr Glu Phe Phe
                245                 250                 255

Ala Lys Arg Gln Leu Val Thr Leu Phe Ser Ala Pro Asn Tyr Cys Gly
            260                 265                 270

Glu Phe Asp Asn Ala Gly Ala Met Met Ser Val Asp Glu Thr Leu Met
        275                 280                 285

Cys Ser Phe Gln Ile Leu Lys Pro Val Glu Lys Lys Lys Ala Ala Asn
    290                 295                 300

<210>  16
<211>  330
<212>  PRT
<213>  Homo sapiens

<400>  16

Met Ser Asp Ser Glu Lys Leu Asn Leu Asp Ser Ile Ile Gln Arg Leu
1               5                   10                  15

Leu Glu Val Gln Gln Ser Arg Pro Gln Lys Asn Val Gln Leu Thr Glu
            20                  25                  30

Asn Glu Ile Arg Gln Leu Cys Leu Lys Ser Arg Glu Ile Phe Leu Ser
        35                  40                  45

Gln Pro Ile Leu Leu Glu Leu Glu Ala Pro Leu Lys Ile Cys Gln Asp
    50                  55                  60

Ile His Gln Gln Tyr Tyr Asp Leu Leu Arg Leu Phe Glu Tyr Gln Gln
65                  70                  75                  80

Phe Pro Pro Glu Ser Asn Tyr Leu Phe Leu Gln Asp Tyr Val Asp Arg
                85                  90                  95

Gln Lys Gln Ser Leu Glu Thr Ile Cys Leu Leu Leu Ala Tyr Lys Ile
            100                 105                 110

Lys Tyr Pro Glu Asn Phe Phe Leu Leu Arg Gln Asn His Glu Cys Ala
        115                 120                 125

Ser Ile Asn Arg Ile Tyr Gln Phe Tyr Asp Glu Cys Lys Arg Arg Tyr
    130                 135                 140

Asn Ile Lys Leu Trp Lys Thr Phe Thr Asp Cys Phe Asn Cys Leu Pro
145                 150                 155                 160

Ile Ala Ala Ile Val Asp Glu Lys Ile Phe Cys Cys His Gln Gln Leu
                165                 170                 175

Ser Pro Asp Leu Gln Ser Met Glu Gln Ile Arg Arg Ile Met Arg Pro
            180                 185                 190

Thr Asp Val Pro Asp Gln Gln Leu Leu Cys Asp Leu Leu Trp Ser Asp
        195                 200                 205

Pro Asp Lys Asp Val Gln Gln Trp Gln Glu Asn Asp Arg Gln Val Ser
    210                 215                 220

Phe Thr Phe Gln Ala Glu Val Val Ala Lys Phe Leu His Lys His Asp
225                 230                 235                 240

Leu Asp Leu Ile Cys Arg Ala His Gln Val Val Glu Asp Gln Tyr Glu
                245                 250                 255

Phe Phe Ala Lys Arg Gln Leu Val Thr Leu Phe Ser Ala Pro Asn Tyr
            260                 265                 270

Cys Gln Glu Phe Asp Asn Ala Gln Ala Met Met Ser Val Asp Glu Thr
        275                 280                 285

Leu Met Cys Ser Phe Gln Ile Leu Lys Pro Ala Asp Lys Asn Lys Gln
    290                 295                 300

Lys Tyr Gln Gln Phe Ser Gln Leu Asn Pro Gln Gln Arg Pro Ile Thr

305                 310                 315                 320

Pro Pro Arg Asn Ser Ala Lys Ala Lys Lys
                325                 330